# EUROPEAN PATENT SPECIFICATION

(11) **EP 0 775 107 B1**
(45) Date of publication and mention of the grant of the patent: **13.10.1999**
(21) Application number: 95930419.7
(22) Date of filing: 28.07.1995
(51) Int. Cl.: C07C 249/12, C07C 251/54

(54) **PREPARATION OF O-(2-HYDROXYALKYL) OXIMES**
VERFAHREN ZUR HERSTELLUNG VON O-(2-HYDROXY)OXIMEN
PREPARATION DE O-(2-HYDROXYALKYLE) OXIMES

(30) Priority: 02.08.1994 DE 4427289
(43) Date of publication of application: 28.05.1997
(73) Proprietor: BASF AKTIENGESELLSCHAFT, 67056 Ludwigshafen (DE)
(72) Inventor: HARREUS, Albrecht, D-67063 Ludwigshafen (DE); GÖTZ, Norbert, D-67547 Worms (DE); RANG, Harald, D-67122 Altrip (DE); HARTMANN, Horst, D-67459 Böhl-Iggelheim (DE); MOHR, Jürgen, D-67269 Grünstadt (DE); GEHRER, Eugen, D-67069 Ludwigshafen (DE); LAUTH, Günter, D-23562 Lübeck (DE)
(86) International application number: EP9503001
(87) International publication number: WO9604238

(56) References cited:
- EP-A- 0 655 437
- US-A- 3 965 177
- CHEMICAL ABSTRACTS, vol. 68, no. 11, 11 March 1968, Columbus, Ohio, US; abstract no. 49175, J. WOLF ET AL. page 4747 ; cited in the application & PL,A,53 525
- CHEMICAL ABSTRACTS, vol. 73, no. 7, 17 August 1970, Columbus, Ohio, US; abstract no. 35231, J. WOLF ET AL. page 316 ; & PL,A,59 077
- R. KLAUS ET AL. 'ACS Symposium Series 443, 1991, Chapt. 18, p. 226-235' , AMERICAN CHEMICAL SOCIETY , WASHINGTON cited in the application see page 231, scheme 9

## Description

The present invention relates to a process for the preparation of O-(2-hydroxyalkyl)oximes of the general formula I 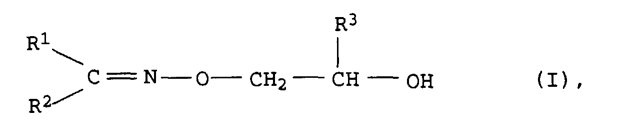 in which R¹ and R² each stand for an alkyl group having from 1 to 10 carbon atoms, or form, together with the carbon atom to which they are attached, a 5-membered to 7-membered cycloalkyl radical, and R³ denotes an alkyl group having from 1 to 10 carbon atoms.

O-(2-hydroxyalkyl)oximes have great significance as intermediates for plant protectants (cf eg, the prior German Application DE-A 44 15 887).

Furthermore, it is well known that it is possible to cause reaction of acetone oxime with ethylene carbonate in toluene in the presence of potassium fluoride to produce O-(2-hydroxyethyl)acetone oxime (cf. Amer. Chem. Soc. Symposium Series 443 page 231(1991)).

Furthermore, a process for the preparation of bis[O-(2-hydroxyethyl)dimethyl-glyoxime starting from dimethylglyoxime and ethylene carbonate is described in J. Polymer Sci., 10, page 3408 (1972).

In accordance with the earlier application EP-A 655,437, aldoximes and ketoximes with unsubstituted or C₁-C₈-alkyl-substituted ethylene or propylene carbonate can be hydroxyalkylated in the presence of catalytic amounts of an N-alkylated, stable organic base or of pyridine substituted by a secondary amine.

It was the object of the invention to provide a more economical process for the preparation of O-(2-hydroxyalkyl)oximes I.

Accordingly, we have found a process for the preparation of O-(2-hydroxyalkyl)-oximes of the formula I, wherein a ketoxime of the formula II 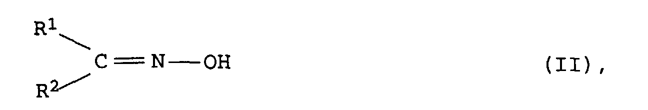 is caused to react with a carbonate of the formula III 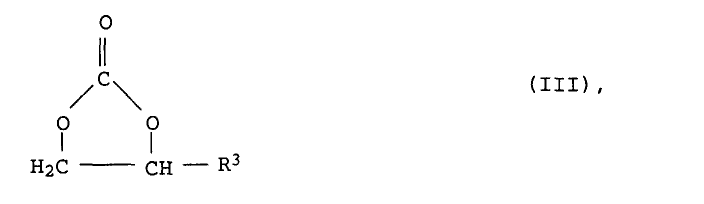 in the presence of a catalyst.

The following literature citations "Houben-Weyl" refer to Houben-Weyl, Methoden der Organischen Chemie, 4th ed., Thieme Verlag, Stuttgart.

Preferred process products I are those which can be produced by this process variant those are preferred in which R¹ and R² stand for C₁-C₄ alkyl groups and primarily C₁-C₃ alkyl groups or form, together with the carbon atom to which they are attached, a cyclopentyl or cyclohexyl ring, and in particular those in which R¹ and R² stand for methyl and/or ethyl or form, together with the carbon atom to which they are attached, cyclohexyl. R³ preferably stands for a C₁-C₄ alkyl group, particularly for ethyl and more particularly for methyl. Most particularly preferred compounds I are those in which R¹, R² and R³ stand for methyl.

The ketoximes II are generally known or are obtainable by known methods, for example, by the reaction of the corresponding ketones with hydroxylamine.

The carbonates III are also generally known or can be produced by known methods (cf. eg, EP-A 543,249).

Reaction of ketoximes II with carbonates IV in the presence of a catalyst:

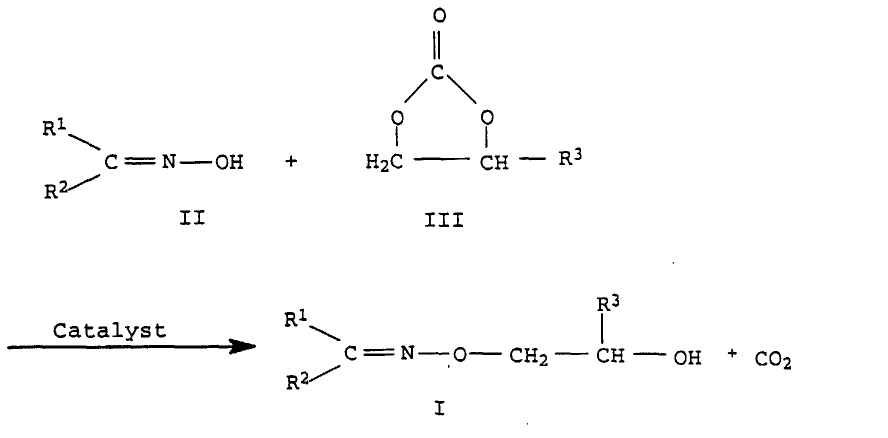

The molar ratio of carbonate III to ketoxime II is usually from 1:1 to 10:1 and in particular from 4:1 to 7:1.

Suitable catalysts, used either alone or in the form of a mixture, are
α) alkali metal salts, alkaline earth metal salts, and ammonium salts (NH₄⁺), primarily the potassium salts and also the sodium salts' with inorganic or organic anions such as alkali metal halides, hydroxides, carbonates, hydrogen carbonates, alcoholates, and alkali metal salts of organic monocarboxylic acids. By way of example, there may be mentioned potassium fluoride, potassium iodide, potassium hydroxide, potassium carbonate, potassium hydrogen carbonate, potassium methylate, potassium ethylate, potassium tertbutylate, and potassium acetate;
β) ammonium salts and preferably phosphonium salts having at least one organic group in the cation and containing inorganic or organic anions, primarily the halides, hydrogen carbonates, and acetates of such phosphonium salts in particular those containing three C₁-C₄ alkyl and/or phenyl groups, such as tri-n-butylphosphonium acetate and triphenylphosphonium chloride;
ξ) phosphines having at least one but preferably three C-organic radicals, in particular those selected from the group comprising C₁-C₁₀ alkyl, C₆-C₁₅-aryl, C₆-C₁₅ alkylaryl, and/or C₇-C₁₀-arylalkyl, such as, primarily tri-n-butylphosphine and triphenylphosphine; or
δ) triethylamine.

Preferred catalysts are those which are mentioned under (α) and (δ). Of the (α) group, the potassium salts are preferably used, especially potassium fluoride or potassium hydrogen carbonate.

The catalyst, primarily the inorganic alkali metal salts and the alkali metal salts of carboxylic acids, can be bonded to a support, if desired. Examples of suitable supports are aluminum oxide, silica gel, and kieselguhr. The amount of catalyst in these supported catalysts is usually from 10 to 50 wt%.

The catalysts are usually employed in an amount of from 5 to 50 mol%, preferably from 5 to 30 mol%, and more preferably from 5 to 10 mol%, based on the ketoxime II.

The reaction can be carried out in a single, preferably aprotic, organic solvent or in a solvent mixture. However, the process is preferably carried out without the use of a solvent, in which case an excess of the carbonate III can, in particular, serve as reaction medium.

Suitable solvents are for example alkylbenzenes, such as, in particular, toluene and also the xylenes, furthermore dialkylketones such as methyl ethyl ketone, halobenzenes such as chlorobenzene, and ethers such as 1,4-dioxane.

The amount of solvent used is usually from 1 to 9 times and in particular from 2 to 7 times the weight of the ketoxime II. lf the amount of solvent exceeds 9 times the weight of the ketoxime II, conversion and yield usually fall.

The reaction is preferably carried out at temperatures of from 80°C to 150°C and mostly from 100°C to 140°C and pressures of from 0.5 to 1 bar and especially under standard pressure.

The addition of a conventional phase transfer catalyst usually leads to an increase in reaction rate and conversion. However, for simple processing, it is preferred not to use a phase transfer catalyst.

Examples of phase transfer catalysts are quaternary ammonium or phosphonium salts, preferably tetraalkylammonium, tetraalkylphosphonium, trialkylbenzylammonium, or trialkylbenzylphosphonium salts, especially triethylammonium, tributylbenzylammonium, and tetrabutylammonium chlorides, bromides, and hydrogen sulfates, and also tributylhexaphosphonium bromide.

The phase transfer catalyst is usually employed in an amount of from 0.5 to 2 mol% and preferably from 0.7 to 1 mol%, based on the ketoxime II.

The reaction can be carried out batchwise or, preferably, continuously.

The reaction times are normally from 8 to 24 h but mostly from 8 to 16 h.

The isolation of the reaction product I takes place by methods known per se, preferably by distillation.

The distillation residues of the crude mixture contain substantially unconverted carbonate III and the catalyst.

In a preferred embodiment of the process of the invention the process is carried out by replenishing the ketoxime II and the carbonate III at a rate equal to the rate of consumption thereof, following the destillation of the product I from the reaction mixture, and the reaction is then repeated.

It has been found that in up to 20 successive reactions carried out in this manner in the same reactor, the yield of the compound I falls inappreciably and the ratio of the isomers I and Ia remains approximately the same.

The reaction frequently also produces, as by-product, the regio-isomeric compound Ia 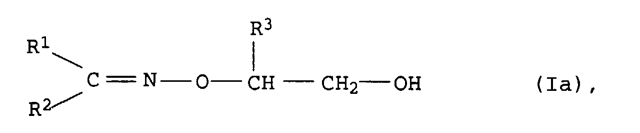 which is usually obtained in amounts of up to 10 %, based on I. The two isomeric compounds I and Ia are normally the first overheads occurring during distillation of the crude product. lf desired, the isomer Ia can be substantially separated from the compound I by methods known per se, preferably by fractional distillation.

The end products I are important intermediates for crop protection agents, especially for herbicides of the cyclohexenone type (cf. DE-A 44 15 887).

### Examples

### Example 1

### Preparation of O-(2-hydroxypropyl)propanone oxime

585 g (8 mol) of acetone oxime, 4080g (40 mol) of propylene carbonate, and 80 g (0.8 mol) of potassium hydrogen carbonate in 200g of toluene were caused to react for a period of 8 h at 130°C. There followed fractional distillation of the reaction mixture. The fraction of desired product consisting of O-(2-hydroxypropyl)propane-2-one oxime and O-(2-hydroxy-1-methylethyl)propan-2-one oxime distilled over at 70- 75°C/20-30 mbar in a percentage ratio of I:Ia of 92:8.

Following cooling of the distillation residues, toluene, acetone oxime, and propylene carbonate were replenished to the aforementioned quantities and the reaction was then repeated. Following further 10 reactions of the type just described, an average was taken over all 12 reactions. The yield of O-(2-hydroxyalkyl) oximes was found to be 90 % based on acetone oxime.

### Example 2

### Preparation of O-(2-hydroxypropyl)propanone oxime

The process was carried out as in Example 1 but without the addition of toluene. Starting from 344 g (4.7 mol) of acetone oxime, 2420 g (23.5 mol) of propylene carbonate, and 47 g (0.47 mol) of potassium hydrogen carbonate there was obtained, in a total of 10 reaction cycles carried out, in each case, for a period of 8 h at 130°C, a mixture consisting of O-(2-hydroxypropyl)propane-2-one oximeand O-(2-hydroxy-1-methylethyl)propane-2-one oxime in a percentage ratio of I:Ia of 92:8 and in a yield of 89%.

### Example 3

### Preparation of O-(2-hydroxypropyl)propanone oxime

The process was carried out as in Example 1 but with the addition of 1086 g of toluene. Starting from 585 g (8 mol) of acetone oxime, 2450 g (24 mol) of propylene carbonate, and 80 g (0.8 mol) of potassium hydrogen carbonate there was obtained, in a total of 5 reaction cycles carried out, in each case over a period of 8 h at 125-130°C, a mixture consisting of 0-(2-hydroxypropyl)propane-2-one oxime and O-(2-hydroxy-1-methylethyl)propan-2-one oxime in a percentage ratio of I:Ia of 92:8 and at a yield of 89 %.

### Example 4

### Preparation of O-(2-hydroxypropyl)propane-2-one oxime

292.5 g (4.0 mol) of acetone oxime, 2040 g (20 mol) of propylene carbonate and 100 g (1.0 mol) of triethylamine were introduced into a stirred apparatus and stirred at an oil bath temperature of 130°C for 8 hours. After cooling, distillation was carried out (bubble-cap tray column, 60 cm long, 30 mm in diameter). 80 g of triethylamine was recovered. The distillation residue, which consisted to the extent of 90% of unconsumed propylene carbonate, was able to be used for the next reaction with acetone oxime.
Yield (b.p. 9 mbar = 46°C): 85%.

## Claims

1. A process for the preparation of O-(2-hydroxyalkyl) oximes of the general formula 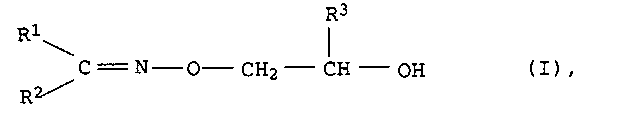 in which R¹ and R² each stand for an alkyl group having from 1 to 10 carbon atoms or form, together with the carbon atom to which they are attached, a 5-membered to 7-membered cycloalkyl radical, and R³ denotes an alkyl group having from 1 to 10 carbon atoms, wherein a ketoxime of the general formula II 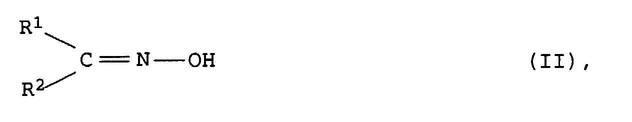 is caused to react with a carbonate of the general formula III 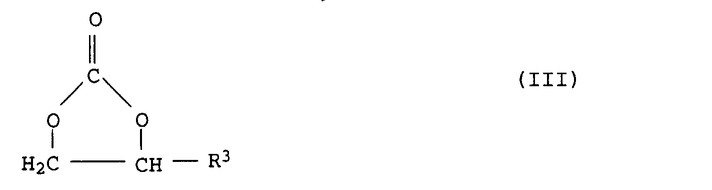 in the presence of a catalyst selected from the group consisting of
α) alkali metal salts, alkaline earth metal salts and ammonium salts (NH₄⁺);
β) ammonium salts and preferably phosphonium salts having at least one organic group in the cation and containing inorganic or organic anions;
γ) phosphines having at least one but preferably three C-organic radicals; or
δ) triethylamine or
mixtures thereof.

2. A process as defined in claim 1, wherein the ketoxime II used is acetone oxime, butanone oxime, or cyclohexanone oxime.

3. A process as defined in claim 1, wherein the ketoxime II used is acetone oxime.

4. A process as defined in any of claims 1 to 3, wherein the starting material is a compound III in which R³ denotes methyl.

5. A process as defined in any of claims 1 to 4, wherein potassium hydrogen carbonate is used as catalyst.

6. A process as defined in any of claims 1 to 4, wherein triethylamine is used as catalyst.

7. A process as defined in claim 1, wherein II is reacted with III without a solvent.

## Patentansprüche

1. Verfahren zur Herstellung von 0-(2-Hydroxyalkyl)oximen der allgemeinen Formel 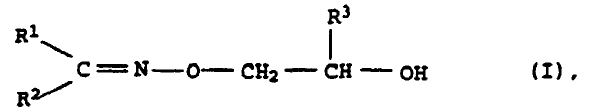 wobei R¹ und R² jeweils für eine Alkylgruppe mit 1 bis 10 Kohlenstoffatomen stehen oder zusammen mit dem sie tragenden Kohlenstoffatom einen 5- bis 7-gliedrigen Cycloalkylrest bilden und R³ eine Alkylgruppe mit 1 bis 10 Kohlenstoffatomen bedeutet, bei dem man ein Ketoxim der allgemeinen Formel II 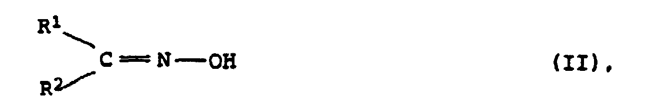 mit einem Carbonat der allgemeinen Formel III 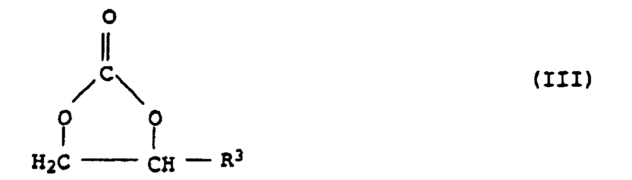 in Gegenwart eines Katalysators aus der Gruppe
α) Alkalimetall-, Erdalkalimetall- und Ammoniumsalze (NH₄⁺),
β) Ammonium- und vorzugsweise Phosphoniumsalze mit mindestens einer organischen Gruppe im Kation und mit anorganischen oder organischen Anionen,
γ) Phosphine mit mindestens einem, vorzugsweise aber drei C-organischen Resten oder
δ) Triethylamin oder
deren Mischungen
umsetzt.

2. Verfahren nach Anspruch 1, bei dem man als Ketoxim II Acetonoxim, Butanonoxim oder Cyclohexanonoxim einsetzt.

3. Verfahren nach Anspruch 1, bei dem man als Ketoxim II Acetonoxim einsetzt.

4. Verfahren nach einem der Ansprüche 1 bis 3, bei dem man von einer Verbindung III ausgeht, in der R³ für Methyl steht.

5. Verfahren nach einem der Ansprüche 1 bis 4, bei dem man als Katalysator Kaliumhydrogencarbonat einsetzt.

6. Verfahren nach einem der Ansprüche 1 bis 4, bei dem man als Katalysator Triethylamin einsetzt.

7. Verfahren nach Anspruch 1, bei dem man die Umsetzung von II mit III ohne Lösungsmittel vornimmt.

## Revendications

1. Procédé pour la préparation d'O-(2-hydroxyalkyl) oximes de formule générale 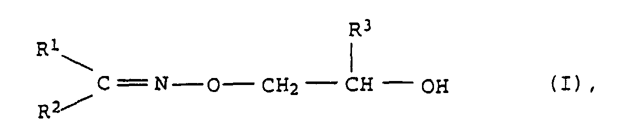 où R¹ et R² désignent chacun un groupe alkyle ayant de 1 à 10 atomes de carbone ou forment, conjointement avec l'atome de carbone auquel ils sont fixés un radical cycloalkyle à 5 à 7 chaînons, et R³ représente un groupe alkyle ayant de 1 à 10 atomes de carbone, procédé dans lequel on fait réagir une cétoxime de formule générale II 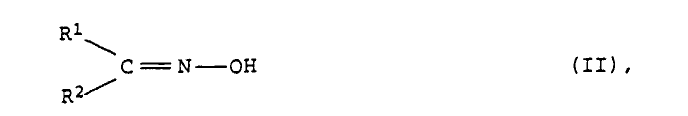 avec un carbonate de formule générale III 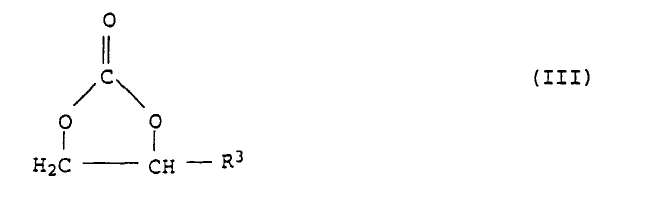 en présence d'un catalyseur choisi dans le groupe consistant en
α) des sels de métal alcalin, des sels de métal alcalino-terreux et des sels d'ammonium (NH₄⁺),
β) des sels d'ammonium et, de préférence, des sels de phosphonium ayant au moins un groupe organique dans le cation et contenant des anions inorganiques ou organiques,
γ) des phosphines ayant au moins un, mais de préférence trois, radicaux organiques carbonés,
δ) de la triéthylamine ou
leurs mélanges.

2. Procédé selon la revendication 1, dans lequel la cétoxime II utilisée est de l'acétone oxime, de la butanone oxime ou de la cyclohexanone oxime.

3. Procédé selon la revendication 1, dans lequel la cétoxime II utilisée est l'acétone oxime.

4. Procédé selon l'une quelconque des revendications 1 à 3, dans lequel la matière première est un composé III dans lequel R³ désigne un radical méthyle.

5. Procédé selon l'une quelconque des revendications 1 à 4, dans lequel de l'hydrogénocarbonate de potassium est utilisé comme catalyseur.

6. Procédé selon l'une quelconque des revendications 1 à 4, dans lequel de la triéthylamine est utilisée comme catalyseur.

7. Procédé selon la revendication 1, dans lequel II est mis à réagir avec III sans solvant.
